Europäisches Patentamt

European Patent Office

Office européen des brevets

(11) Veröffentlichungsnummer: **0 268 813**
**B1**

(12) **EUROPÄISCHE PATENTSCHRIFT**

(45) Veröffentlichungstag der Patentschrift:
05.12.90

(51) Int. Cl.⁵: **A61K 31/17, A61K 9/16**

(21) Anmeldenummer: 87115170.0

(22) Anmeldetag: 16.10.87

(54) Verfahren zur Herstellung eines bindemittelfreien tablettierfähigen Celiprololhydrochlorid-Granulates.

(30) Priorität: 24.10.86 DE 3636209

(43) Veröffentlichungstag der Anmeldung:
01.06.88 Patentblatt 88/22

(45) Bekanntmachung des Hinweises auf die Patenterteilung:
05.12.90 Patentblatt 90/49

(84) Benannte Vertragsstaaten:
AT BE CH DE ES FR GB GR IT LI LU NL SE

(56) Entgegenhaltungen:
EP-A- 0 009 858
DE-A- 2 702 504
DE-A- 3 410 380

(73) Patentinhaber: CL PHARMA AKTIENGESELLSCHAFT,
St. Peter-Strasse 25, A-4021 Linz(AT)

(84) Benannte Vertragsstaaten: BE CH DE ES FR GB GR IT LI LU NL SE AT

(72) Erfinder: Schlünken, Heinrich, Dr., Boschweg 1c,
A-4020 Linz(AT)

(74) Vertreter: Kunz, Ekkehard, Dr., Chemie Holding AG Patentwesen St. Peter-Strasse 25, A-4021 Linz(AT)

# Beschreibung

Die Erfindung betrifft ein Wirbelschichtverfahren zur Gewinnung eines tablettierfähigen, bindemittelfreien Celiprololhydrochlorid-Granulates sowie die Verwendung eines nach diesem Verfahren erhaltenen Granulates zur Herstellung von festen Arzneiformen, die Celiprolol-Hydrochlorid enthalten.

Celiprolol-Hydrochlorid (3-(3-Acetyl-4-(3-tert. butylamino-2-hydroxypropoxy)-phenyl)-1,1-diäthyl-harnstoff-Hydrochlorid), ein in der Humanmedizin verwendeter besonders bewährter Betablocker zur Behandlung verschiedener Herzerkrankungen, kann z.B. nach den AT-Psen 334 385 oder 380 232 hergestellt werden und wird zur oralen Applikation bevorzugt in Tablettenform verabreicht.

Celiprolol-Hydrochlorid liegt nach der Synthese üblicherweise als sehr feines Pulver, bei dem mehr als 50 % eine Korngröße unter 40 µm besitzt vor, wobei die Fließfähigkeit des Pulvers gänzlich fehlt. Dieses Pulver ist daher für eine Direkttablettierung nicht geeignet.

Da Celiprolol-Hydrochlorid bei der oralen Applikation in verhältnismäßig großen Dosen verabreicht werden muß, sind die Möglichkeiten, durch Vermischen des Pulvers mit größeren Mengen an Hilfsstoffen wie Fließregulierungsmitteln zu direkt tablettierbaren Pulvermischungen zu gelangen, beschränkt, da so hergestellte Arzneiformen zu voluminös und daher für die orale Applikation nicht oder nur eingeschränkt geeignet sind.

Zur Überwindung dieser Schwierigkeiten wird Celiprolol-Hydrochlorid bisher beispielsweise in einer Feuchtgranulierung mit Kollidon als Bindemittel zu einem verpressbaren Granulat verarbeitet. Hier ist der Nachteil, daß infolge des hohen erforderlichen Bindemittelgehaltes zur Erreichung von akzeptablen Zerfallszeiten der Zusatz von größeren Mengen an Sprengmittel wie Formalin-Kasein oder Ac-Di-Sol (quervernetzte Natriumcarboxymethylzellulose) erforderlich ist. Gegen Formalin-Kasein sind jedoch in letzter Zeit toxikologische Bedenken aufgetreten und Ac-Di-Sol kann zu Verfärbungen der Tablettenoberfläche führen.

Es ist bereits bekannt, daß man Feuchtgranulierungen unter Zuhilfenahme eines Bindemittels in der Wirbelschicht durchführen kann. In der EP-A 49 143 ist ein Verfahren beschrieben, bei dem eine Bindemittellösung auf den fluidisierten Wirkstoff (L-Ascorbat) bei Wirbelschichttemperaturen von bis zu 80°C aufgesprüht wird, wobei ein tablettierfähiges Granulate entsteht. Eine Übertragung dieses Verfahrens auf Celiprolol-Hydrochlorid hat sich als unbraucher erwiesen, da einerseits die Verwendung von Bindemitteln wiederum zu den oben angeführten Nachteilen geführt hat und andererseits festgestellt werden mußte, daß die Wirbelschicht bei höheren Temperaturen zusammenbricht, was zum Festbacken des Wirbelschichtguts an der Wand des Wirbelschichtgranulators führte. Aus diesem Grund ist auch eine Trocknung von Celiprolol-Hydrochlorid in der Wirbelschicht nicht möglich.

Überraschenderweise wurde nun gefunden, daß sich Celiprolol-Hydrochlorid-Pulver mit einem Wirbelschichtverfahren in vollkommen bindemittelfreie Granulate, die sich zu Tabletten mit ausgezeichneten Eigenschaften verpressen lassen, überführen läßt, wenn man die Agglomeration bei niedrigen Temperaturen durch Einsprühen von Wasser ohne Zugabe eines Bindemittels durchführt und das entstandene Granulat nach dem Entfernen aus der Wirbelschicht trocknet.

Gegenstand der Erfindung ist demnach ein Verfahren zur Herstellung eines bindemittelfreien, zu festen Arzneiformen verpressbaren Granulates von Celiprolol-Hydrochlorid, welches darin besteht, daß man eine vorgegebene Menge an Celiprolol-Hydrochlorid, bei dem mehr als 50 % eine Korngröße unter 40 µm und höchstens 10 % eine Korngröße von über 100 µm besitzen, in einem Wirbelschichtgranulator vorlegt, durch Einströmen von Zuluft mit einer Temperatur bis maximal 30° C eine Wirbelschicht aufbaut und durch Einsprühen von destilliertem Wasser in das Wirbelbett ein rieselfähiges Granulat mit einem Wassergehalt von 20 - 40 Gew. % herstellt, bei dem weniger als 10 % eine Korngröße unter 63 µm besitzen, und dieses nach dem Austrag bei Temperaturen von 30 -60° C trocknet.

Für die Ausführung des erfindungsgemäßen Verfahrens eignen sich handelsübliche Wirbelschichtgranulatoren. Solche Wirbelschichtgranulatoren bestehen im wesentlichen aus einem Materialbehälter, in den das Fluidisiergas seitlich oder von unten einströmt, wobei sowohl die Strömungsgeschwindigkeit als auch die Temperatur des einströmenden Fluidisiergases regelbar ist. Weiters verfügen diese Geräte über einen oder mehrere Sprühköpfe in Form von Einstoff- oder Zweistoffdüsen, über die reine Flüssigkeiten und Lösungen mittels einer Pumpe mit einstellbarer Sprühgeschwindigkeit in den Materialbehälter eingesprüht werden können und die so angeordnet sind, daß die zerstäubte Flüssigkeit oder Lösung direkt in die Wirbelschicht hineinströmt. Ein mechanisches Rühr- oder Zerhackerwerk auf dem Behälterboden oder ein Rüttelwerk soll bei Bedarf für einen zusätzlichen Mischeffekt sorgen.

Als Fluidisiergas kann man in einfachster Weise und ohne Nachteil für die Qualität des Wirkstoffes Luft verwenden. Es eignen sich aber selbstverständlich auch andere Gase, die gegenüber der Wirksubstanz inert sind, wie beispielsweise Stickstoff, dafür.

Das für die Granulierung verwendete Celiprolol-Hydrochlorid-Pulver, das zu über 50 % Anteile mit einer Korngröße unter 50 µm aufweist, soll zu maximal 10 % Anteile mit einer Korngröße über 100 µm enthalten. Sollte es Anteile von über 250 µm enthalten, muß es vor der Wirbelschichtgranulierung gemahlen werden.

Zur praktischen Durchführung des Verfahrens wird eine dem jeweiligen Wirbelschichtgranulator angepaßte Menge an Celiprolol-Hydrochlorid im Materialbehälter vorgelegt, durch Einströmen des Fluidisiergases bei maximal 30° C eine Wirbelschicht aufgebaut und anschließend Wasser eingesprüht.

Die einzusprühende Wassermenge hängt von der vorgelegten Menge an Celiprolol-Hydrochlorid, von der Feuchte und Temperatur des Fluidisiergases, der Dauer der Wirbelschichtgranulierung und dem gewünschten Wassergehalt des Granulates ab. Sie wird so gewählt, das das erhaltene feuchte Granulat einen Wassergehalt von etwa 20 - 40 Gew. % aufweist und beträgt etwa 20 und 60 Gew. % der vorgelegten Menge an Celiprolol-Hydrochlorid. Die eingesprühte Wassermenge und die Temperatur des Fluidisiergases, die höchstens 30° C betragen darf, vorteilhafterweise jedoch Raumtemperatur besitzt, werden so aufeinander abgestimmt, daß ein Granulat mit oben angegebenen Eigenschaften erhalten wird, wobei selbstverständlich der während des Aufsprühens verdampfende Anteil der Wassermenge berücksichtigt werden muß.

Die Ermittlung des Endpunktes der Befeuchtung erfolgt durch visuelle Beobachtung der Agglomeration sowie der Bestimmung des Wassergehaltes einer Probe nach K. Fischer.

Gemäß einer bevorzugten Ausführungsform der vorliegenden Erfindung werden die Parameter der Wirbelschichtgranulierung so aufeinander abgestimmt, daß das erhaltene Granulat nach dem Austrag aus dem Wirbelschichtgranulator einen Wassergehalt von etwa 25 - 30 Gew. % besitzt. Bevorzugterweise beträgt die eingesprühte Wassermenge 25–35 Gew. % der vorgelegten Celiprolol-Hydro chlorid-Menge.

Wenn sich ein Granulat mit den gewünschten Eigenschaften gebildet hat, wird dieses kontinuierlich oder diskontinuierlich aus dem Wirbelschichtgranulator ausgetragen und einer Feuchtsiebung durch ein Sieb mit einer Maschenweite von beispielsweise 2,0 mm unterworfen.

Die Trocknung des feuchten Granulates muß außerhalb des Wirbelschichtgranulators erfolgen, da sich das Granulat bei einer Temperatur oberhalb von 30° C an der Behälterwand anlegt und unter 30° C wieder in ein Pulver zerfällt. Sie kann nach dem Austrag aus dem Wirbelschichtgranulator mit oder ohne Vakuum auf alle in der Galenik üblichen Arten erfolgen. Vorzugsweise wird das Granulat auf Hordenblechen bei Temperaturen von 30 - 60° C, bevorzugt 38 - 40° C, getrocknet. Die Trocknungszeit ist abhängig von der Temperatur und kann in einem gewissen Bereich schwanken. Eine bevorzugte Trocknungszeit ist 10 - 20 Stunden. Auf diese Weise erhält man Granulate, deren Restwassergehalt 2 %, bezogen auf das Gesamtgewicht, nicht übersteigt.

Das erhaltene Granulat weist zu weniger als 10 % eine Korngröße von unter 63 µm auf. Gemäß einer bevorzugten Ausführungsform werden Granulate erhalten, bei denen weniger als 0,5 % eine Korngröße über 1 000 µm und weniger als 5 % eine Korngröße von unter 63 µm besitzen. Besonders bevorzugt sind Granulate mit den oben genannten Grenzen, bei denen 70 % der Anteile eine Korngröße zwischen 160 und 800 µm besitzen.

Die Vorteile des vorliegenden Verfahrens liegen in der Einfachheit des Verfah rens und in der Erhöhung der Produktqualität des erhaltenen Granulates. Die Verwendung von Wasser anstelle von bindemittelhaltiger Granulierflüssigkeit führt zu Granulaten, die aus reiner Wirksubstanz bestehen, wobei die sich als ungünstig erwiesene Verwendung von Bindemitteln überraschenderweise vermieden werden kann. Diese Granulate lassen sich entweder direkt oder gegebenenfalls nach Zusatz weiterer pharmazeutisch verträglicher Hilfs- oder Wirkstoffe zu festen Arzneiformen mit ausgezeichneten galenischen Eigenschaften wie hoher mechanischer Festigkeit und niedriger Zerfallszeit verpressen ohne daß eine Interaktion zwischen Bindemittel und Wirk- und Hilfsstoffen befürchtet werden muß.

Solche festen Arzneiformen umfassen Presslinge jeder Größe und Form, wie Tabletten, Dragees, Tabletten- und Drageekerne, Mehrschichttabletten oder ganz kleine Komprimate, die zum Einfüllen in Hart- oder Weichgelatinekapseln bestimmt sind, und dergleichen. Diese Arzneiformen können mit den üblichen Schutzhüllen und Überzügen versehen werden und können geschmacksverbessernde Stoffe enthalten.

Beispiel:

5 000 g Celiprolol-Hydrochlorid werden in einem Wirbelschichtgranulator (WSG 5 der Fa. Glatt, Deutschland) vorgelegt. Durch Einströmen von Luft von Raumtemperatur (15 - 25° C) wird die Wirbelschicht aufgebaut. Mit einer Schlauchpumpe werden 1 500 ml destilliertes Wasser bis zum Einsetzen einer sichtbaren Agglomeration mit einer Sprühgeschwindigkeit von ca. 70 ml/min. eingesprüht.

Alle 2 Minuten wird für 30 Sekunden das Rüttelwerk eingeschaltet.

Nach 30 Minuten kann man durch visuelle Beobachtung den Beginn der Agglomerationsbildung erkennen. Dies ist erfahrungsgemäß der beste Zeitpunkt zum Abbrechen der Wirbelschichtgranulierung. Das feuchte Granulat besitzt nach dem Austrag aus dem Wirbelschichtgranulator von 29 Gew.%.

Nach einer Feuchtsiebung auf einer Frewitt-Siebmaschine durch ein Sieb mit einer Maschenweite von 2,0 mm wird das Granulat im Trockenschrank auf Hordenblechen bei einer Temperatur von 39° C 15 Stunden getrocknet. Der Restwassergehalt beträgt danach 1,6 %.

Die Untersuchung der Korngrößenverteilung lieferte folgende Werte:

über 1 00 µm - %
800 µm - 1000 µm 1,5 %
500 µm - 800 µm 22,3 %
250 µm - 500 µm 35,3 %
160 µm - 250 µm 20,7 %
100 µm - 160 µm 10,7 %
63 µm - 100 µm 5,9 %
unter 63 µm 3,6 %
Das Granulat besitzt eine ausgezeichnete Fließfähigkeit.

**Patentansprüche**

1. Verfahren zur Herstellung eines bindemittelfreien, zu festen Arzneiformen verpressbaren Granulates von Celiprolol-Hydrochlorid, dadurch gekennzeichnet, daß man eine vorgegebene Menge an Celiprolol-Hydrochlorid, bei dem mehr als 50 % eine Korngröße unter 40 µm unter höchstens 10 % eine Korngröße von über 100 µm besitzen, in einem Wirbelschichtgranulator vorlegt, durch Einströmen von Zuluft bei einer Temperatur von max. 30°C eine Wirbelschicht aufbaut, durch Einsprühen von destilliertem Wasser in das Wirbelbett ein rieselfähiges Granulat mit einem Wassergehalt von 20 - 40 Gew. % herstellt, bei dem weniger als 10 % eine Korngröße von unter 63 µm besitzen, und dieses nach dem Austrag bei Temperaturen von 30 bis 60° C trocknet.

2. Verfahren gemäß Anspruch 1, dadurch gekennzeichnet, daß das rieselfähige Granulat vor dem Trocknen einen Wassergehalt von 25 - 30 Gew. % besitzt.

3. Verfahren gemäß den Anspruch 1 und 2 dadurch gekennzeichnet, daß die eingesprühte Wassermenge 20 bis 60 Gew.% der vorgelegten Menge an Celiprolol-Hydrochlorid beträgt.

4. Verfahren gemäß Anspruch 3, dadurch gekennzeichnet, daß die eingesprühte Wassermenge 25 - 35 Gew. % der vorgelegten Menge an Celiprolol-Hydrochlorid beträgt.

5. Verfahren gemäß einem der Ansprüche 1 bis 4, dadurch gekennzeichnet, daß man ein Granulat aufbaut, bei dem weniger als 0,5 % eine Korngröße über 1 000 µm und weniger als 5 % eine Korngröße unter 63 µm besitzen.

6. Verfahren gemäß einem der Ansprüche 1 bis 6, dadurch gekennzeichnet, daß man ein Granulat aufbaut, bei dem 70 % der Anteile eine Korngröße zwischen 160 und 800 µm besitzen.

7. Verfahren gemäß einem der Ansprüche 1 bis 6, dadurch gekennzeichnet, daß man das Granulat bis zu einem Restwassergehalt von weniger als 2 Gew.% trocknet.

8. Verfahren gemäß Ansprüchen 1 bis 7, dadurch gekennzeichnet, daß man das Granulat im Trockenschrank bei Temperaturen von 38 - 40° C 10 - 20 Stunden trocknet.

9. Verwendung eines nach Ansprüchen 1 bis 8 hergestellten Granulates zur Herstellung fester Arzneiformen von Celiprolol-Hydrochlorid, dadurch gekennzeichnet, daß man das Granulat mit in der Galenik üblichen Hilfe- und Zusatzstoffen und gegebenenfalls einem oder mehreren Wirkstoffen vermischt und auf übliche Weise verpreßt.

## Claims

1. Process for the preparation of binder-free granules of celiprolol hydrochloride which can be compressed to give solid medicament forms, characterized in that a predetermined amount of celiprolol hydrochloride, over 50% of which have a particle size below 40 µm and not more than 10% have a particle size above 100 µm is initially placed in 3 fluidized bed granulator, a fluidized bed is formed by passing in additional air at a temperature not higher than 30°C and, by spraying distilled water into the fluidized bed, free-flowing granules having a water content of 20 – 40% by weight and less than 10% of which have a particle size below 63 µm are prepared and these granules are dried at temperatures from 30 to 60°C after they have been discharged.

2. Process according to Claim 1, characterized in that the free-flowing granules have a water content of 25 – 30% by weight before being dried.

3. Process according to Claim 1 and 2, characterized in that the amount of water sprayed in is 20 to 60% by weight of the amount of celiprolol hydrochloride initially taken.

4. Process according to Claim 3, characterized in that the amount of water sprayed in is 25 – 35% by weight of the amount of celiprolol hydrochloride initially taken.

5. Process according to one of, Claims 1 to 4, characterized in that granules in which less than 0.5% have a particle size above 1,000 µm and less than 5% have a particle size below 63 µm are formed.

6. Process according to one of Claims 1 to 6, characterized in that granules in which 70% of the fraction have a particle size between 160 and 800 mm, are formed.

7. Process according to one of Claims 1 to 6, characterized in that the granules are dried to a residual water content of less than 2% by weight.

a. Process according to Claims 1 to 7, characterized in that the granules are dried for 10 – 20 hours at temperatures of 38 – 40°C in a drying cabinet.

9. The use of granules prepared in accordance with Claims 1 to 8 for the preparation of solid medicament forms of celiprolol hydrochloride, characterized in that the granules are mixed with auxiliaries and additives customary in pharmaceutical production and, if appropriate, with one or more further active compounds, and are compressed in a customary manner.

## Revendications

1. Procédé de préparation d'un granulé de chlorddhydrate de céliprolol , exempt de liant apte à être compressé sous forme de comprimés solides. selon lequel on introduit dans un appareil de granulation à lit fluidisé une quantité donnée de chlorhydrate de céliprolol présentant une fraction de plus de 50% ayant une granulométrie supérieure à 100 µm, on réalise par introduction d'air à une température de 30°C au plus un lit fluidisé, on prépare un granulé apte à s'écouler, ayant une proportion en masse d'eau comprise entre 20 et 40%. par pulvérisation d'eau distillée dans le lit fluidisé dans lequel une fraction d'au plus de 10% a une granulométrie inférieure à 63 µm, et en ce qu'on le sèche après introduction à une température de 30 à 60°C.

2. Procédé selon la revendication 1, caractérisé en ce que le granulé coulable comprend un taux d'humidité de 25 à 30% en masse avant séchage.

3. Procédé selon la revendication 1 ou 2, caractérisé en ce que la quantité d'eau pulvérisée représente 20 à 60% en masse de la quantité de chlorhydrate de céliprolol utilisée.

4. Procédé selon la revendication 3, caractérisé en ce que la quantité d'eau pulvérisée représente 25 à 35% en masse de la quantité de chlorhydrate de céliprolol utilisée.

5. Procédé selon l'une des revendications 1 à 4, caractérisé en ce qu'on prépare un granulé comportant une fraction de moins de 0,5% ayant une granulométrie supérieure à 1000 μm et une fraction de moins de 5% ayant une granulométrie inférieure à 63 μm.

6. Procédé selon l'une des revendiquions 1 à 5, caractérisé en ce qu'on prépare un granulé comportant une fraction de 70% avent une granulométrie comprise entre 160 et 800 μm.

7. Procédé selon l'une des revendications 1 à 6, caractérisé en ce qu'on sèche le granulé jusqu'à une teneur en eau de moins de 2% en masse.

8. Procédé selon les revendications 1 à 7. caractérisé en ce qu'on sèche le granulé dans une armoire de séchage à une température de 38 à 40°C pendant 10 à 20 heures.

9. Utilisation d'un granulé obtenu selon l'une des revendications 1 à 8 pour préparer des médicaments solides de chlorhydrate de céliprolol, caractérisé en ce qu'on mélange le granulé avec des additifs ou suivants usuels dans le processus galénique et le cas échéant avec une ou plusieurs substances actives et que l'on comprime de manière usuelle.